# NEW EUROPEAN PATENT SPECIFICATION

(11) **EP 0 889 710 B2**
(45) Date of publication and mention of the opposition decision: **26.10.2005**
(45) Mention of the grant of the patent: 27.02.2002
(21) Application number: 97914438.3
(22) Date of filing: 25.03.1997
(51) Int. Cl.: A61J 3/07

(54) **IMPROVEMENTS IN OR RELATING TO ENCAPSULATION**
VERBESSERUNG AN EINKAPSELUNG
AMELIORATIONS EN MATIERE D'ENCAPSULATION

(30) Priority: 26.03.1996 GB 9606371
(43) Date of publication of application: 13.01.1999
(73) Proprietor: BioProgress Technology Limited, March, Cambridgeshire PE15 0AX (GB)
(72) Inventor: BROWN, Malcolm David, Mundford, Norfolk IP26 5DS (GB)
(74) Representative: Hill, Justin John
(86) International application number: PCT/GB1997/000838
(87) International publication number: WO 1997/035537

(56) References cited:
- WO-A-91/04017
- WO-A-95/17153
- WO-A-97/34806
- GB-A- 758 642
- US-A- 2 288 327
- US-A- 2 936 263
- US-A- 3 198 740
- US-A- 3 892 905
- US-A- 3 999 358
- US-A- 4 115 292
- US-A- 4 154 636

## Description

### Field of Invention

This invention concerns encapsulation and relates to a method of encapsulation, encapsulation apparatus and the resulting capsules.

### Background to the Invention

The provision of water soluble and digestible capsules containing pharmaceutical or cosmetic preparations is well established. Typically oils are encapsulated in gelatin shells designed to release their contents when subjected to immersion in water or exposure to digestive juices. These oils include dietary enhancement substances or, in the case of cosmetic preparations, fragrant oils for release into bath water. A substantial industry has been built up around this principle, based primarily on the use of gelatin for the capsule shells. This gelatin is derived from the bones and skins of animals.

With concern for the environment and animal welfare and fear of animal related diseases such as Bovine Spongiform Encephalopathy (BSE) increasing, the number of individuals adopting a serious stance on the use of animals and animal-derived products in food substances and cosmetic applications has risen dramatically. As a result, the sales of such gelatin capsules are very limited among such individuals. There exists therefore the need for the provision of a suitable substitute for gelatin in order to provide water soluble or digestible capsules which are not derived from animals. Whilst this is a desirable aim, few materials lend themselves to such use and the machinery currently creating such capsules has been specifically designed to suit the properties of gelatin. As a result, a change in material for the capsule shell requires a redesign of the machinery if it is to have the capability of efficiently producing capsules from the replacement material. It is a change in material and the commensurate machine requirements needed to enable successful processingwhichthis invention addresses.

US 3 198 740 disclose a method of forming packets from water soluable films of polyvinyl alcohol in which a preferred method of forming the packets is to apply to the film immediately prior to the packet forming step by wiping thereon or by other well known means enough water to render the film tackey and sealing the two layers of film together by pressure alone without added heat while the film is still in the tackey state.

WO 91/04017 discloses a method of enrobing in which a unitary core is enrobed between two films, the films being conditioned for enrobing by heating. The method is principally concerned with gelatine based films but other films which have been shown to be useful include films defined principally by polyvinyl alcohol and alginates among others.

US 3 198 740 disclose a method of forming packets from film in which water to render the film tackey is wiped on the film immediately prior to the packet forming process.

WO 91/04017 discloses a method of enrobing in which a unitary core is enrobed between two films, the films being conditioned for enrobing by heating.

### Summary of the Invention

In one aspect the invention provides. A method of making capsules by means of encapsulation of a supplied substance, comprising supplying two films of like material said material being a gelatin substitute capable of deforming elastically at least when partially solvated; applying solvent to at least one of the films to cause partial solvation of the material surface and consequent greater flexibility of the film material; supplying substance to be encapsulated between the films to deform the film material in its more flexible state, into suitably shaped capsule portions which can adhere to each other as a result of the adhesive action of the partially solvated surface(s); and sealing the capsule portions together without the application of heat, encapsulating the supplied substance, to form a capsule..

In the encapsulation unit, the substance to be encapsulated is supplied between the films, the films are formed, typically by a moulding process, Into suitably shaped capsule portions which can adhere to each other as a result of the adhesive action of the partially solvatedsurface(s), and which seal together encapsulating the supplied substance, forming a capsule.

Conventional gelatin encapsulation relies upon heat as the mechanism for sealing the two portions of the shell together to enclose the contents. The capsules made by this invention do not use heat as the primary means of securing the two portions of the capsule together, but instead make use of the adhesive effects manifested when suitable films are partially solvated at their surface.

The films may be of a range of different materials. Suitable materials soluble in water (hot or cold) include polyvinyl alcohol (PVA), alginate, hydroxypropyl methyl cellulose and polyethylene oxide. In this case it is simply necessary to apply water at a suitable temperature to the film or films to cause partial solvation. The resulting capsules release their contents when immersed in water or exposed to digestive juices and thus lend themselves to such uses as the release of fragrant oil in a bath or the release of dietary supplements after ingestion.

Non-water soluble film materials may also be used, such as polycaprolactone and gelatinized starch based materials. In this case it is necessary to apply a suitable solvent such as N-methyl pyrrolidone rather than water to at least one film surface to induce partial solvation. The partial solvation of such films causes them to soften, enabling them to take up the internal dimensions of a mould used to create a capsule. Capsules made from films which are biodegradable but not water soluble release their contents as a result of microbial action instead of immersion in water, and can find use in agricultural and industrial applications.

The currently preferred film material is PVA, preferably plasticised with glycerin. Suitable films are commercially available in a range of different grades, types and thicknesses. An appropriate film can be readily selected having regard to the intended use, capsule contents and desired capsule properties. For example, PVA film is available in thicknesses ranging between 20 and 1000 microns. For cosmetic applications, good results have been obtained with 80 micron PVA film, eg Hi-Selon (Hi-Selon is a Trade Mark) cold water soluble PVA B9, obtainable from British Traders and Shippers, 429-431 Rainham Road South, Dagenham, Essex.

It is preferred to use film material that becomes more flexible when in partially solvated conditions as this assists capsule formation. PVA has this property.

Instead of using pre-formed films, the films may be formed during the encapsulation method, eg by being cast from solution.

In practising the invention it is appropriate to use two films of like material. The films should be chemically alike but need not be identical in terms of factors such as grade, thickness etc.

The solvent is selected having regard to the film material, and is conveniently water in the case of water soluble materials. The solvent should be applied in an appropriate amount, either in isolation or as part of a formulation containing materials such as thickening and/or wetting agents, to cause a suitable degree of partial solvation of the film material surface: this can be readily determined by experiment.

The solvent is preferably applied just prior to encapsulation at an appropriate location to obtain optimum speed of capsule production.

The solvent can be applied in a variety of diffferent ways, including by atomisation such as in the form of a spray or jet, by dipping, electrostatic coating, roller, air knife or Meyer bar, or with a sponge. The currently preferred technique is by means of a gravure or flexo printing process as this enables ready control and regulation of the amount and uniformity of solvent application.

Solvent may be applied to one or both films as appropriate.

A vacuum is conveniently applied during capsule portion formation to assist deformation of the film material.

The invention may be used to encapsulate a wide range of substance in the form of solids, liquids or gases. The invention may, for example, be used to encapsulate all of the substances currently encapsulated in gelatin, such as drugs, vitamins, powders, oils, cosmetic preparations, drug delivery systems, paint etc. A typical cosmetic application is encapsulation of bath oils to produce capsules intended to be used in the bath, where the capsule shell dissolves releasing the oil into the bath water.

The capsules may have a variety of different sizes and shapes, usually determined by the shape of the mould employed. Typically the capsules are spherical or oval, but more elaborate forms eg based on fruit, animal or abstract shapes may be produced, usually for cosmetic applications.

In a further aspect the invention provides. Apparatus for making capsules by means of encapsulating an applied substance, comprising means for supplying two films of like material to an encapsulation unit, said material being capable of deforming elastically at least when partially solvated; an encapsulation unit; and means for supplying a solvent for the film material to at least one of the firms upstream of the encapsulation unit, wherein, in use, the means for supplying solvent is operable to apply solvent to at least one of the films to cause partial solvation of the material surface and consequent greater flexibility of the film material, and wherein said encapsulation unit comprises means for supplying a substance to be encapsulated between the films to deform the film material in its more flexible state into suitably shaped capsule portions which can adhere to each other as a result of the adhesive action of the partially solvated suxface(s), said apparatus being devoid of means for heating the film material.

The encapsulation unit may be based on those used in conventional apparatus currently used for gelatin encapsulation. In typical conventional apparatus, two separate ribbons of gelatin film are first produced by pouring heated liquid gelatin at a controlled rate onto the peripheral faces of two cylinders each rotating about a horizontal axis. The liquid gelatin cools on the cylinders and forms two ribbons which are fed from opposed sides to an encapsulation unit.

The encapsulation unit typically comprises a pair of similar moulding drums. The outer cylindrical face of each drum is formed with a plurality of indentations of desired form, eg hemispherical, arranged in a series of axially extending rows with, say, 5 or 6 indentations in each row. The drums are supported in side by side relationship, with a small gap, there between, and are arranged for coordinated rotation in opposed directions (the left hand drum clockwise, and the right hand drum anticlockwise). A similar arrangement may be used in the present invention. Means for applying a vacuum inside the drums are conveniently included, to help pull the partially solvated films into the indentations and so assist capsule portion formation.

The encapsulation unit typically also comprises a reservoir of the substance to be encapsulated, eg bath oil, and an associated supply arrangement adapted simultaneously to supply a plurality of metered doses (one for each indentation in a row on the moulding drums) of the substance to the moulding drums at predefined time intervals. The arrangement may employ syringe pumps or the like. Again, a similar arrangement may be used in the present invention.

The metered doses are initially supplied to a heated injection segment located above the nip between the moulding drums, and including a row of a plureality of injectors aligned with the rows of indentations in the drums. A similar arrangement may again be used in the present invention although there is no need for the injection segment to be heated.

In use in conventional encapsulation, two gelatin ribbons are formed and fed over appropriate guide rollers etc to pass below the injection segment and into the nip between the counter-rotating rollers. Metered doses of the substance to be encapsulated are injected into the nip in synchronism with the drum rotation. The heating segment also acts to heat the gelatin films, which has the effect of making the films capable of sealing to each other and also makes the films more elastic. As the doses of substance are injected between the heated films, the films deform to line the indentations, forming series of pairs of opposed capsule halves containing the substance. The pairs of capsule halves are brought together, sealed and cut from the gelatin ribbons on continued rotation of the drums, thus forming capsules containing the substance. A typical production rate is one row of capsules every 2 seconds. Instead of cutting the capsules from the ribbons, they may be left integral with the ribbons. The resulting capsules are collected below. The capsules are then typically tumbled in a hot air dryer and then kept in a controlled humidity environment for about 2 days to stabilise the capsules. The capsules are then ready for use or sale.

As noted above, the present invention may use an encapsulation unit generally as described. It is not necessary for the injection segmentto be heated, as the present invention does not rely on heating for sealing, as in the prior art, so processing costs may be reduced somewhat and faster processing may be possible.

Conventional encapsulation apparatus would, of course, also need modification by removal of the gelatin ribbon formation equipment and substitution of equipment for supplying (and possibly also forming) the films of material used in the present invention. In a simple case this could just be a pair of spindles each for receiving a roll of the film, to be fed to the encapsulation unit in known manner.

A further necessary modification is addition of means for applying solvent to one or both of the films, preferably located just upstream of the encapsulation unit. As noted above these means could be a spray or jet arrangement, a bath for dipping, an electrostatic coating unit, a roller, an air knife, a Meyer bar, a sponge etc. Preferably, however, a gravure or flexo printing unit is used.

Means for applying a vacuum within the moulding drums are conveniently also incorporated.

The invention also covers capsules formed in accordance with a method or by use of apparatus in accordance with the invention.

The invention also includes within its scope a capsule having a shell comprising material capable of adhering to and sealing with itself when in partially solvated condition.

In a preferred aspect the invention covers a capsule having a shell comprising polyvinyl alcohol.

The invention will further be described, by way of illustration, in the following Example and with reference to the accompanying drawing, in which:

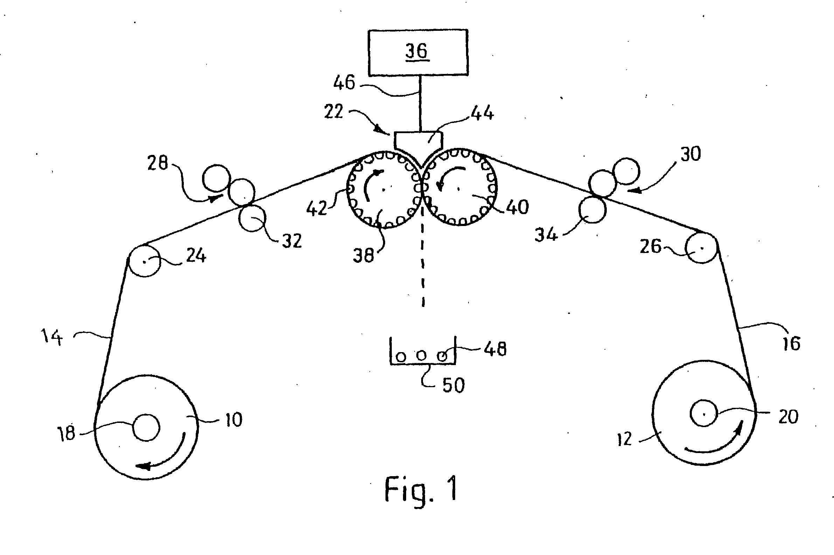

Figure 1 is a schematic illustration of one embodiment of apparatus in accordance with the invention.

### Detailed Description of the Embodiment

The apparatus illustrated in Figure 1 comprises two rolls 10, 12 of film 14, 16 of like material rotatably supported on spindles 18, 20, with associated means (not shown) for feeding film from the rolls to an encapsulation unit 22. The films first pass over respective support rollers 24, 26 and then through respective flexographic printing units 28, 30 with associated backing rollers 32, 34 for supply to a surface of the film, in an adjustable manner, of accurately metered quantities of solvent for the film material. In an experimental apparatus, laboratory scale narrowflexographic headsfrom RK Print Coat Instruments Limited, Litlington, Royston, U. K. were used for this purpose.

The encapsulation unit 22 is based on the encapsulation unit of conventional apparatus, as discussed above, and comprises a reservoir containing the substance to be encapsulated and an associated supply arrangement for supplying metered doses of the substance. The reservoir and supply arrangement are represented schematically at 36.

The encapsulation unit further comprises a pair of similar moulding drums 38, 40. The outer cylindrical face of each drum is formed with a plurality of hemispherical indentations 42 arranged in a series of axially extending rows with 6 indentations in each row. Vacuum means (not shown) may optionally be included for applying a vacuum inside the drums to assist deformation of the film material. The drums are supported in side by side relationship with a small gap therebetween, and are arranged for coordinated rotation in opposed directions (the left hand drum 38 clockwise, and the right hand drum 40 anticlockwise). An injection segment 44 is located above the nip between the moulding drums to receive substance from reservoir and supply arrangement 36, as illustrated schematically by line 46. Injection segment 44 includes an array of 6 injectors (not shown) aligned with the rows of indentations in the drums.

In use, film 14, 16 is supplied at an appropriate rate to the encapsulation unit 22, passing over support rollers 24, 26 and through printing units 28, 30 where solvent is applied to the film surface in appropriate amount. The films then pass below the injection segment 44 and into the nip between drums 38, 40 which are counter-rotating at an appropriate speed. Metered doses of the substance to be encapsulated are injected into the nip from injection segment 44 in synchronism with the drum rotation. As the doses of substance are injected between the films, the films deform to line the indentations 42 of one row in each of the drums, possibly assisted by application of a vacuum, forming a series of 6 pairs of opposed capsule halves containing the substance. On continued rotation of the drums the pairs of capsule halves are brought together and seal because of the adhesive effect caused by partial solvation of the film surface, producing a row of surface-containing capsules which are cutfrom the films. One row of 6 capsules is produced approximately every 2 seconds. The resulting capsules 48 are collected in a tray 50, and the waste film remaining is disposed of. The capsules are dried and stabilised in generally conventional manner.

### Example

Using the apparatus of Figure 1 encapsulation of a typical bath oil cosmetic product was carried out using Hi-Selon cold water soluble plasticised polyvinyl alcohol (B9) film, 80 micron thick, with partial solvation carried out by application of water. This resulted in production of good quality capsules, suitable for cosmetic use.

## Claims

1. A method of making capsules by means of encapsulation of a supplied substance, comprising supplying two films (14, 16) of like material said material being a gelatin substitute capable of deforming elastically at least when partially solvated; applying solvent to at least one of the films (14, 16) to cause partial solvation of the material surface and consequent greater flexibility of the film material; supplying substance to be encapsulated between the films (14, 16) to deform the film material (14, 16) in its more flexible state into suitably shaped capsule portions which can adhere to each other as a result of the adhesive action of the partially solvated surface(s); and sealing the capsule portions together without the application of heat, encapsulating the supplied substance, to form a capsule (48).

2. A method according to claim 1, wherein a vacuum is applied during capsule portion formation to assist deformation of the film material (14, 16).

3. A method according to claim 1 or 2, wherein the film material (14, 16) is selected from polyvinyl alcohol, aginate, hydroxyprogyl methyl cellulose, polyethylene oxide, polycaprolactone and gelatinized starch based materials.

4. A method according to claim 3, wherein the film material (14, 16) is polyvinyl alcohol and the solvent is water.

5. A method according to any one of the preceding claims, wherein the solvent is applied just prior to encapsulation.

6. A method according to any one of the preceding claims, wherein the solvent is applied by means of a gravure or flexo printing process.

7. Apparatus for making capsules by means of encapsulating an applied substance, comprising means (18, 20) for supplying two films (14, 16) of like material to an encapsulation unit (22), said material being capable of deforming elastically at least when partially solvated; an encapsulation unit (22); and means (28, 30) for supplying a solvent for the film material (14, 16) to at least one of the films (14, 16) upstream of the encapsulation unit (22) wherein, in use, the means for supplying solvent is operable to apply solvent to at least one of the films (14, 16) to cause partial solvation of the material surfaces and consequent greater flexibility of the film material, and wherein said encapsulation unit comprises means for supplying a substance to be encapsulated between the films (14, 16) to deform the film material (14, 16) in its more flexible state into suitably shaped capsule portions which can adhere to'each other as a result of the adhesive action of the partially solvated surface(s), said apparatus being devoid of means for heating the film material (14, 16).

8. Apparatus according to claim 7, wherein the encapsulation unit (22) comprises a pair of counter-rotation moulding drums (38, 40) and an associated arrangement (36) for coordinated supply of substance to be encapsulated.

9. Apparatus according to claim 8, including means for applying a vacuum inside the moulding drums (38, 40).

## Patentansprüche

1. Verfahren zur Herstellung von Kapseln durch Einkapselung einer zugeführten Substanz, umfassend:
- Zuführen von zwei Filmen (14, 16) aus gleichem Material, wobei das Material ein Gelatineersatz ist, das zumindest wenn es partiell solvatisiert ist, fähig ist, sich elastisch zu verformen; Aufbringen von Lösungsmittel auf mindestens einen der Filme (14, 16), um eine partielle Solvatisierung der Materialoberfläche und daraus folgend eine größere Flexibilität des Filmmaterials zu bewirken; Zuführen der Substanz, die eingekapselt werden soll, zwischen die Filme (14, 16) unter Verformen des Filmmaterials (14, 16) in seinem flexibleren Zustand zu passend geformten Kapselteilen, die als Resultat der Klebewirkung der partiell solvatisierten Oberfläche(n) aneinander haften können; und Versiegeln der Kapselteile miteinander ohne Anwendung von Wärme, wobei eine Einkapselung der zugeführten Substanz unter Bildung einer Kapsel (48) erfolgt.

2. Verfahren nach Anspruch, 1, wobei während der Formung des Kapselteils Vakuum angelegt wird, um eine Verformung des Filmmaterials (14, 16) zu unterstützen.

3. Verfahren nach Anspruch 1 oder Anspruch 2, wobei das Filmmaterial (14, 16) unter Polyvinylalkohol, Alginat, Hydroxypropylmethylcellulose, Polyethylenoxid, Polycaprolacton und Materialien auf der Basis von gelatinisierter Stärke ausgewählt wird.

4. Verfahren nach Anspruch 3, wobei das Filmmaterial (14, 16) Polyvinylalkohol ist und das Lösungsmittel Wasser ist.

5. Verfahren nach einem der vorangehenden Ansprüche, wobei das Lösungsmittel unmittelbar vor der Einkapselung aufgebracht wird.

6. Verfahren nach einem der vorangehenden Ansprüche, wobei das Lösungsmittel mittels eines Tiefdruckvertahrens oder eines Flexodruckverfahrens aufgebracht wird.

7. Apparatur zur Herstellung von Kapseln durch Einkapselung einer aufgebrachten Substanz, umfassend: Mittel (18, 20) zur Zuführung von zwei Filmen (14, 16) aus gleichem Material zu einer Einkapselungseinheit (22), wobei das Material zumindest wenn es partiell solvatisiert ist, fähig ist, sich elastisch zu verformen; eine Einkapselungseinheit (22) und Mittel (28, 30) zur Zuführung eines Lösungsmittels für das Filmmaterial (14, 16) zu mindestens einem der Filme (14, 16) stromaufwärts der Einkapselungseinheit (22), wobei das Mittel zur Zuführung von Lösungsmittel bei Verwendung funktionsfähig ist, um Lösungsmittel auf mindestens einen der Filme (14, 16) aufzubringen, um eine partielle Solvatisierung der Materialoberfläche und daraus folgend eine größere Flexibiliät des Filmmaterials zu bewirken, und wobei die Einkapselungseinheit Mittel zur Zuführung einer Substanz, die eingekapselt werden soll, zwischen die Filme (14, 16) unter Verformen des Filmmaterials (14, 16) in seinem flexibleren Zustand zu passend geformten Kapselteilen umfasst, die als Resultat der Klebewirkung der partiell solvatisierten Oberfläche(n) aneinander kleben können, wobei die Apparatur keine Mittel zum Erwärmen des Filmmaterials (14, 16) aufweist.

8. Apparatur nach Anspruch 7, wobei die Einkapselungseinheit (22) ein Paar gegenläufiger Formwalzen (38, 40) und eine zugeordnete Vorrichtung (36) zur koordinierten Zuführung der Substanz, die eingekapselt werden soll, umfaßt.

9. Apparatur nach Anspruch 8, die Mittel zum Anlegen eines Vakuums im Innern der Formwalzen (38, 40) umfaßt.

## Revendications

1. Procédé pour la réalisation de capsules par encapsulation d'une substance fournie, comprenant la fourniture de deux films (14, 16) de matière semblable, ladite matière étant un substitut de gélatine, pouvant se déformer élastiquement au moins lorsqu'ils sont partiellement dissous ; l'application d'un solvant à au moins un des films (14, 16) pour produire une dissolution partielle de la surface de la matière et obtenir par conséquent une plus grande souplesse de la matière des films ; la fourniture d'une substance à encapsuler entre les films (14, 16) pour déformer la matière des films (14, 16) dans son état le plus souple en parties de capsule de forme appropriée qui peuvent adhérer l'une à l'autre comme résultat de l'action adhésive de la surface ou des surfaces partiellement dissoute(s) ; et la fermeture mutuelle des parties de capsule sans application de chaleur, la substance fournie étant ainsi encapsulée, pour former une capsule (48).

2. Procédé selon la revendication 1; dans lequel un vide est appliqué pendant la formation des parties de capsule, pour aider à la déformation de la matière des films (14, 16).

3. Procédé selon la revendication 1 ou 2, dans lequel la matière des films (14, 16) est choisie parmi : alcool polyvinylique, alginate, hydroxypropyl méthyl cellulose, oxyde de polyéthylène, polycaprolactone et matières à base d'amidon gélatinisé.

4. Procédé selon la revendication 3, dans lequel la matière des films (14, 16) est l'alcool polyvinylique et le solvant est l'eau.

5. Procédé selon une quelconque des revendications précédentes, dans lequel le solvant est appliqué juste avant l'encapsulation.

6. Procédé selon une quelconque des revendications précédentes, dans lequel le solvant est appliqué au moyen d'un procédé de gravure ou de flexo impression.

7. Appareil pour réaliser des capsules par encapsulation d'une substance fournie, comprenant des moyens (18, 20) de fourniture de deux films (14, 16) de matière semblable à une unité d'encapsulation (22), ladite matière étant capable de se déformer élastiquement au moins quand elle est partiellement dissoute ; une unité d'encapsulation (22) ; et des moyens (28, 30) pour l'application d'un solvant de la matière des films (14, 16) à au moins un des films (14, 16) en amont de l'unité d'encapsulation (22), dans lequel, en utilisation, les moyens pour l'application du solvant sont adaptés pour appliquer le solvant à au moins un des films (14, 16) pour provoquer la dissolution partielle de la surface de la matière et par conséquent une meilleure flexibilité de la matière des films, et dans lequel l'unité d'encapsulation comprend des moyens pour fournir une substance destinée à être encapsulée entre deux films (14, 16) pour déformer la matière des films (14, 16) dans son état le plus flexible en parties de capsules de forme appropriée qui peuvent adhérer l'une à l'autre comme résultat de l'action adhésive de la surface ou des surfaces partiellement dissoute(s), ledit appareil ne comportant pas de moyen de chauffage de la matière des films (14, 16).

8. Appareil selon la revendication 7, dans lequel l'unité d'encapsulation (22) comprend deux tambours de moulage tournant en sens inverse (38, 40), et un dispositif associé (36) pour coordonner la fourniture de substance à encapsuler.

9. Appareil selon la revendication 8, incluant des moyens d'application d'un vide à l'intérieur des tambours de moulage (38, 40).
